# EUROPÄISCHE PATENTANMELDUNG

(11) **EP 3 453 362 A1**
(43) Veröffentlichungstag der Anmeldung: **13.03.2019**
(21) Anmeldenummer: 17190595.3
(22) Anmeldetag: 12.09.2017
(51) Int. Cl.: A61D 3/00, A61B 6/04, A61G 13/02, A61G 13/04

(54) **GROSSTIERBEHANDLUNGSTISCH**

(71) Anmelder: Kober, Bernd, 68723 Schwetzingen (DE); Kowalewski, Tim, 63589 Linsengericht (DE)
(72) Erfinder: Kober, Bernd, 68723 Schwetzingen (DE); Kowalewski, Tim, 63589 Linsengericht (DE)
(74) Vertreter: Kramer Barske Schmidtchen Patentanwälte PartG mbB

(57) **Zusammenfassung**

Die vorliegende Erfindung betrifft einen Großtierbehandlungstisch (5) mit einem verfahrbaren Grundgestell (10), das in Längsachsenrichtung gesehen ein Vorderende und ein Hinterende aufweist und zumindest im Bereich des Vorderendes ein geteiltes Fahrgestell mit einem rechten Fahrgestellteil (25) und einem von dem rechten Fahrgestellteil (25) beabstandeten linken Fahrgestellteil (26) umfasst. Eine Hubvorrichtung (15) hat ein unteres Auflager (33), mit dem die Hubvorrichtung (15) im Bereich des Hinterendes des Grundgestells (10) angeordnet ist, und ein oberes Auflager (34). Eine Tischplatte (20) ist mit dem oberen Auflager (34) der Hubvorrichtung (15) derart verbunden, dass die Tischplatte (20) einen frei auskragenden Tischbereich bildet und in einer Seitenansicht das Grundgestell (10), die Hubvorrichtung (15) und die Tischplatte (20) einen C-förmigen Umriss haben. Durch das geteilte Fahrgestell (25, 26) und die C-Förmige Ausgestaltung ist die Tischplatte (20) über einen Tisch eines üblichen Behandlungs- oder Diagnosegerätes wie z.B. ein Computertomographiegerät bewegbar. Zusätzlich oder alternativ kann ein Strahlerkopf eines Linearbeschleunigers um den auskragenden Tischbereich herum rotieren.

## Beschreibung

### Technisches Gebiet

Die vorliegende Erfindung betrifft allgemein mobile Behandlungstische für die Veterinärmedizin, die auch für Großtiere wie z.B. Pferde verwendbar sind und bei denen das Tier auf einer höhenverstellbaren Tischplatte zu Behandlungszwecken gelagert wird.

### Hintergrund

Großtierbehandlungstische der eingangs genannten Bauart werden für die universelle Diagnose und Behandlung von Klein- und Großtieren benötigt. Die Tiere werden hierbei auf einer Tischplatte gelagert, um Zugang zum Tierkörper für die Durchführung von Diagnose- und Behandlungstätigkeiten zu bekommen.

So wird zum Beispiel unter der Bezeichnung Haico Pferde OPTisch PRO 3 ein Großtierbehandlungstisch vertrieben, dessen Tischplatte in der Höhe verstellbar und in vier Richtungen kipp- und neigbar ist. Die Tragkraft beträgt 1.300 kg. Seitlich der Tischplatte sind Auflageverbreiterungen anbringbar. In einem Mittelteil der Tischplatte ist eine Aussparung vorgesehen, um die Bewegungsfreiheit für einen Operateur zu erhöhen. Alle Auflageteile können entfernt werden. Ein Kopfteil kann seitlich verschoben werden. Dieser Behandlungstisch ist aufgrund der zentralen Hubvorrichtung nicht mit anderen bekannten Behandlungstischen kombinierbar. Damit lässt dieser bekannte Großtierbehandlungstisch insbesondere keine Durchführung von radioonkologischen Behandlungen mittels Computertomographie oder Linearbeschleunigern mit rotierbarem Strahlerkopf zu.

Ein beweglicher Untersuchungstisch für die Humanmedizin, der nicht für Großtiere ausgelegt und geeignet ist, aus der DE 10 2010 064 209 A1 bekannt. Hier ist ein beweglicher Untersuchungstisch offenbart, der eine mobile Plattform zum Tragen eines Menschen, einen unter der mobilen Plattform befestigten und zum Tragen der mobilen Plattform verwendeten Träger sowie eine unter der mobilen Plattform angeordnete Führungsschiene umfasst. Der Untersuchungstisch umfasst außerdem eine Transportvorrichtung, die zum Antrieb des Trägers zur Bewegung auf der Führungsschiene verwendet werden kann. Insbesondere soll dieser Untersuchungstisch in einem Notfall erlauben, die mobile Plattform auf dem Untersuchungstisch von der Transportvorrichtung schnell zu trennen und nach Behebung des Notfalls in die Ausgangsposition zurückzubringen.

Ein ebenfalls aus der Humanmedizin bekannter mobiler Untersuchungstisch wird unter der Bezeichnung Roesys X-Mobil durch die Firma Medizintechnik St. Egidien GmbH vertrieben. Auch dieser mobile Untersuchungstisch ist nur für maximale Patientengewichte von 250 kg und allein deswegen nicht für Großtiere geeignet.

Es besteht zunehmend Bedarf an Großtierbehandlungstischen, die nicht nur zu Untersuchungs- und Behandlungszwecken einsetzbar sind, sondern auch Operationen, radioonkologische Behandlungen sowie Strahlentherapien von Großtieren zulassen.

Das der Erfindung zugrundeliegende technische Problem besteht somit darin, einen Großtierbehandlungstisch bereitzustellen, der die Durchführung von Operationen und die Untersuchung von Großtieren mit bekannten Röntgen- und Strahlentherapiegeräten wie insbesondere Linearbeschleunigern oder auch Computertomographiegeräten erlaubt.

### Zusammenfassung der Offenbarung

Das genannte technische Problem wird durch einen Großtierbehandlungstisch gelöst, der ein fahrbares Grundgestell, eine Hubvorrichtung und eine Tischplatte umfasst. Das Grundgestell weist in Längsachsenrichtung ein Vorderende und ein Hinterende auf und umfasst zumindest im Bereich des Vorderendes ein geteiltes Fahrgestell mit einem rechten Fahrgestellteil und einem von dem rechten Fahrgestellteil beabstandeten linken Fahrgestellteil, um einen vom Vorderende her zugänglichen Freiraum unterhalb der Tischplatte zu schaffen. Die Hubvorrichtung umfasst ein unteres Auflager, mit dem die Hubvorrichtung im Bereich des Hinterendes des Grundgestells angeordnet ist, und ein oberes Auflager. Die Tischplatte ist mit dem oberen Auflager der Hubvorrichtung derart verbunden, dass sie einen frei auskragenden Tischbereich hat und in einer Seitenansicht das Grundgestell, die Hubvorrichtung und die Tragplatte einen asymmetrischen, im Wesentlichen C-förmigen Umriss bilden. Durch das geteilte Fahrgestell und die asymmetrische Anordnung der Hubvorrichtung ist die Tischplatte über einen vorhandenen Tisch eines üblichen Behandlungs- oder Diagnosegerätes wie z.B. ein Computertomographiegerät bewegbar. Zusätzlich oder alternativ ist durch die erfindungsgemäße Ausgestaltung des Großtierbehandlungstisches ein Strahlerkopf eines Linearbeschleunigers um den auskragenden Tischbereich herum rotierbar.

Der Erfindung liegt der Gedanke zugrunde, die grundlegenden Bestandteile eines Großtierbehandlungstisch trotz der erheblichen Lasten, die aufzunehmen sind, so auszubilden, dass auch ein Großtier in bekannte Röntgen-oder Computertomographiegeräte einbringbar ist, um mit Hilfe dieser Geräte Untersuchungen an dem Großtier vornehmen zu können. Durch die zum Hinterende hin versetzte Anordnung der Hubvorrichtung und die geteilte Fahrgestellausbildung ist der Großtierbehandlungstisch über mit den Geräten festverbundenen Tische verfahrbar. Die dadurch geschaffene C-förmige Formgebung hat sich trotz der hohen aufzunehmenden Traglasten überraschend als geeignet erwiesen. Durch die in Draufsicht zumindest im vorderen Bereich geteilte Ausgestaltung des Fahrgestells ist es zudem erstmals möglich, sogenannte Rotations- und stereotaktische Bestrahlungen mittels Linearbeschleuniger nun auch an Großtieren durchzuführen, da ein Strahlerkopf eines Linearbeschleunigers um das Großtier, das auf dem erfindungsgemäßem Behandlungstisch liegt, um 360° rotiert werden kann. Da die Tischplatte des erfindungsgemäßen Behandlungstisches auf die minimale Höhe des vorhandenen Tisches des Behandlungs- oder Diagnosegeätes abgesenkt werden kann, ist es erstmals möglich, Tiere mit großem Körperumfang, wie beispielsweise Pferde, z.B. mittels eines aufgesetzten Elektronentubus zu bestrahlen

Die Tischplatte ist für die Aufnahme der hohen Traglasten und unter Berücksichtigung der auskragenden Ausgestaltung möglichst steif auszubilden. Vorzugsweise besteht die Tischplatte zumindest zum Teil und/oder im Vorderbereich aus Materialien, die eine große Steifigkeit haben und eine geringe Strahlenabsorption aufweisen. Geeignete Materialien sind z.B. Kohlenstofffaserverstärkte Kunststoffe (CFK).

Da der erfindungsgemäße Großtierbehandlungstisch über Tische bekannter medizinischer Geräte mit dem darauf befindlichen Großtier fahrbar ist, können bisher bekannte Computertomographiegeräte, Röntgengeräte und übliche Linearbeschleuniger erstmals auch bei Großtieren zum Einsatz kommen. Bisher hätte man für den Einsatz derartiger medizinischer Geräte an Großtieren deren feststehende Tische abbauen müssen. Ein Austausch eines Tisches z.B. eines Bestrahlungsgerätes oder Computertomographiegerätes ist allerdings nur mit sehr großem Aufwand möglich und äußerst Kostenintensiv.

Eine beispielhafte Ausführungsform eines erfindungsgemäßen Großtierbehandlungstisches sieht vor, dass die Hubvorrichtung in Längsrichtung gesehen jeweils einen linken Hubteil und einen rechten Hubteil umfasst, zwischen denen ein Freiraum vorhanden ist. Der Freiraum zwischen den beiden Hubteilen erlaubt, dass die Tischplatte über einen vorhandenen Behandlungstisch z. B. eines Computertomographiegerätes verfahrbar ist und zwar so weit, dass die Hubteile rechts und links des vorhandenen Tisches zum Liegen kommen. Demzufolge ist die Tischplatte z.B. sehr tief in einen Gantry eines Computertomographiegerätes hinein verfahrbar.

Vorzugsweise ist bei einer beispielhaften Ausführungsform eines erfindungsgemäßen Großtierbehandlungstisches ein Motor vorhanden, mit dem über ein Getriebe sowohl der rechte als auch der linke Hubteil in der Höhe verstellbar sind. Beispielsweise ist hierzu jeweils ein vorderes Lager der Hubteile über eine gemeinsame Gewindespindel mit einer Gewindebuchse verbunden. Durch Drehen der Gewindespindel verändert sich der Abstand der vorderen Lager zu der Gewindebuchse und entsprechend dieser Abstandsveränderung erfolgt ein Absenken oder Anheben der Hubteile. Vorzugsweise wird die Gewindespindel von einem Elektro- oder Hydraulikmotor gedreht. Vorzugsweise kann zusätzlich eine Handkurbel vorhanden sein, mit der bei Ausfall des Motors eine Höhenverstellung durch einen Bediener per Hand möglich ist.

Eine weitere beispielhafte Ausführungsform eines erfindungsgemäßen Großtierbehandlungstisches sieht vor, dass das rechte und linke Hubteil jeweils einen Scherenmechanismus umfassen. Damit ist eine konstruktiv einfache, kostengünstige und gleichzeitig äußerst tragfähige Ausgestaltung der Hubvorrichtung möglich. So kann der behandelte Veterinär auch am hinteren Tischplattenende bequem sitzen, indem die Beine zwischen den beiden Hubteilen platziert werden.

Zur Verbesserung der Standfähigkeit und/oder der Kippfestigkeit eines Großtierbehandlungstisches gemäß der Erfindung weist das Grundgestell vorzugsweise zumindest am Vorderende eine größere Breite auf als am Hinterende. Gleichzeitig ist die Zugänglichkeit gewisser Körperbereiche des auf der Tischplatte liegenden Großtiers für einen Operateur oder Tierveterinär erleichtert.

Bei einer weiteren beispielhaften Ausführungsform eines erfindungsgemäßen Großtierbehandlungstisches ist die Tischplatte neigbar. Dies ist für bestimmte Untersuchungen und Operationen vorteilhaft.

Die Neigung der Tischplatte ist vorzugswiese dadurch einstellbar, dass ein starres Sperrelement an einem Ende verdrehbar mit dem oberen Auflager im Bereich der Tischrotationsachse und mit dem anderen Ende mit dem Grundgestell verbindbar ist. Durch diese einfache und kostengünstige Ausgestaltung wird beim Absenken der Hubvorrichtung die Tischplatte automatisch um eine Querachse geneigt. Somit ist kein separater Antrieb für die Neigungseinstellung notwendig. Durch die Länge des Sperrelements und die möglichen Verbindungsstellen mit dem Auflager und dem Grundgestell ist auch die maximale Neigung als auch die Neigrate beim Absenken der Tischplatte festlegbar. Die Neigrate gibt an, um wieviel Grad sich die Tischplatte bei einer definierten Absenkhöhe neigt; also z.B. es wird eine Neigung um 10° bei einer Absenkung um 200 mm erzielt.

Vorzugsweise ist das Sperrelement als verschwenkbarer Bügel oder in Form wenigstens eines Stabes ausgebildet, dessen Enden an entsprechenden Stellen am Grundgestell und/oder am oberen Lager steck- oder einhängbar ausgestaltet sind. Damit ist eine kostengünstige und gleichzeitig zuverlässige Neigungseinstellung realisierbar.

Zum Zweck der Neigungseinstellung der Tischplatte ist vorzugsweise das obere Auflager der Hubvorrichtung an einem hinteren oberen Lager mit der Tischplatte um eine Querachse verdrehbar gelagert und an einem vorderen oberen Lager mit der Tischplatte gleitend bzw. abwälzend gelagert ist.

Ferner kann beispielsweise außerdem das untere Auflager der Hubvorrichtung an einem hinteren Lager mit dem Grundgestell um eine Querachse verdrehbar gelagert sein und an einem vorderen Lager auf dem Grundgestell gleitend oder abwälzend gelagert sein. Gleitend gelagert soll hier nicht nur klassische Gleitverbindungen umfassen, sondern auch Roll- oder Wälzlagerungen.

Eine beispielhafte kostengünstige Ausführungsform eines erfindungsgemäßen Großtierbehandlungstisches sieht vor, dass das rechte Fahrgestellteil und das linke Fahrgestellteil jeweils mit Laufrollen versehen sind und vorzugsweise zumindest im Bereich des Vorderendes eine freie Öffnung mit einer lichten Innenweite von mindestens 700 mm definieren. Damit ist ein "Auffahren" des Großtierbehandlungstisches auf bestehende Tische medizinischer Untersuchungs- oder Behandlungsgeräte der hier genannten Art in einfacher Weise ermöglicht.

Vorzugsweise hat das Grundgestell wenigstens im Bereich des Vorderendes eine maximale Höhe über Boden von ungefähr 150 mm.

Um sowohl eine geeignete Arbeitshöhe für einen Veterinär zu schaffen, aber auch um alle denkbaren Behandlungen mit bestehenden medizinischen Geräten durchführen zu können, ist beispielsweise die Tischplatte auf eine Minimalhöhe von ungefähr 700 mm absenkbar und/oder auf eine Maximalhöhe von ungefähr 1400 mm anhebbar. Durch die erfindungsgemäße Ausgestaltung eines Großtierbehandlungstisches ist es auch erstmals möglich, eine Hubvorrichtung vorzusehen, die diese niedrige Absenkung der Tischplatte trotz der hohen Traglast erlaubt. Dies ist insbesondere dann vorteilhaft, wenn bei dem Großtier eine Bestrahlung von Hauttumoren mit Elektronen durchgeführt werden soll. Bei dieser Art von Elektronenbestrahlung wird in aller Regel ein Elektronentubus verwendet, der eine definierte Länge hat. Diese wird direkt unter dem Strahlerkopf befestigt. Die Unterseite des Elektronentubus wird bei der Behandlung direkt auf die Haut des Tieres aufgesetzt. Damit auch Tiere mit großem Bauchumfang, wie zum Beispiel Pferde, entsprechend bestahlt werden können, ist es erstmals möglich, den Abstand zwischen Lagerungsmedium und Strahlerquelle möglichst groß auszugestalten, das heißt, die Tischplatte möglichst tief abzusenken.

Indem das Grundgestell wenigstens im Bereich des Vorderendes beispielsweise eine maximale Höhe über Boden von 150 mm aufweist, ist es möglich, insbesondere einen Strahlerkopf eines üblichen Linearbeschleunigers um den vorkragenden Tischbereich der Tischplatte über 360 Grad um eine Längsachse rotieren zu lassen. Damit sind radioonkologische Behandlungen am Großtier mittels bekannter Linearbeschleuniger möglich, trotz dass dabei der Strahlerkopf während einer Rotation knapp oberhalb des Bodens vorbeistreicht.

Insbesondere ist es also mit einem Großtierbehandlungstisch gemäß der vorliegenden Erfindung möglich, eine sogenannte Rotationsbestrahlung bzw. stereotaktische Bestrahlungen am Tier durchführen zu können, insbesondere auch in Bereichen, die nicht von oberhalb der Tischplatte erreichbar sind.

Bei einer weiteren beispielhaften Ausführungsform der vorliegenden Erfindung weist die Tischplatte des Großtierbehandlungstisches an wenigstens einem Längsrand wenigsten eine Steckmöglichkeit für ein Tischverbreiterungselement und/oder eine Fixiervorrichtung, mit der beispielsweise Gliedmaßen eines Großtieres in gewünschter Position mittels Wendern oder Zuggurten in bestimmter Lage zur Tischplatte gehalten werden können, auf.

Zur Vereinfachung der Konstruktion ist es insbesondere vorteilhaft, wenn zur Erzielung einer Neigung der Tischplatte keine spezielle Drehachse und entsprechende Lager vorgesehen sein müssen. Somit ist es bei einer beispielhaften Ausführungsform der vorliegenden Erfindung zweckmäßig, ein Sperrelement verdrehbar mit dem oberen Lager im Bereich der Tischrotationsachse und dem Grundgestell zu verbinden, so dass bei Absenken der Hubvorrichtung die Tischplatte sich automatisch neigt. Je nach eingestelltem Abstand zwischen dem oberen Drehlager des Sperrelements und dem unteren Drehlager an dem Grundgestell in Verbindung mit der Absenkhöhe des Tisches ist die Tischneigung einstellbar.

Vorzugsweise besteht die Hubvorrichtung aus zwei voneinander in Querrichtung beabstandeten Scherenmechanismen, die insbesondere über einen gemeinsamen Motor und zugeordnetes Getriebe in der Höhe verstellbar sind. Hierzu weisen die beiden Scherenmechanismen vorzugsweise ein oberes Auflager auf, das an einem jeweiligen hinteren Ende des Scherenmechanismus mit der Tischplatte an einer ortsfesten Drehachse drehbar gelagert ist. Ein vorderes Ende des jeweiligen Scherenmechanismus ist mit dem Auflager gleitend verbunden.

Bei einer weiteren bevorzugten Ausführungsform sind die beiden unteren Enden der Scherenmechanismen entsprechend ausgebildet. Das heißt, ein unteres hinteres Ende des Scherenmechanismus ist mit dem Grundgestell drehbar gelagert, ein vorderes unteres Ende ist gleitend auf dem jeweiligen Träger des Grundgestells gelagert. Damit sind zum einen große Tragkräfte ohne weiteres aufnehmbar und trotz dieser großen Lasten kann die Tischplatte in der Höhe verstellt werden. Ferner ist ein Freiraum sowohl zwischen dem Grundgestell bzw. den jeweiligen Grundgestellteilen und den beiden Hubmechanismen geschaffen, so dass ein vollständig freier Zwischenraum unterhalb der Tischplatte zwischen den beiden Scherenmechanismen vorhanden ist, was das Überfahren eines bestehenden Tisches einer Computertomographieanlage oder dergleichen ermöglicht.

Zur bestmöglichen Lagerung unterschiedlich großer Tiere ist vorzugsweise die Tischplatte an wenigstens einem Längsrand und/oder Seitenrand mit wenigstens eine Steckmöglichkeit für ein Tischverbreiterungselement versehen.

In einer weiteren beispielhaften Ausführungsform sind ein oder mehrere Laufrollen motorisch angetrieben, um den Großtierbehandlungstisch leichter bewegen zu können.

### Kurze Beschreibung der Zeichnungen

Im Folgenden sind zur weiteren Erläuterung und zum besseren Verständnis der vorliegenden Erfindung mehrere Ausführungsbeispiele näher beschrieben und erläutert. Es zeigen:
Fig. 1 eine schematische perspektivische Schrägansicht von oben eines erfindungsgemäßen Großtierbehandlungstisches,
Fig. 2 eine Seitenansicht des Großtierbehandlungstisches der Fig. 1,
Fig. 3 eine der Fig. 2 entsprechende Seitenansicht des Großtierbehandlungstisches gemäß der vorliegenden Erfindung mit abgesenkter und geneigter Tischplatte,
Fig. 4 eine schematische perspektivische Ansicht von schräg oben des Großtierbehandlungstisches der Fig. 3,
Fig. 5 eine Explosionsansicht der Tischplatte eines erfindungsgemäßen Großtierbehandlungstisches mit verschiedenen Anbaumöglichkeiten,
Fig. 6 den Behandlungstisch von Fig. 5 mit angebrachten Anbauteilen,
Fig. 7 eine schematische Vorderansicht des in der Fig. 1 gezeigten Ausführungsbeispiels eines erfindungsgemäßen Großtierbehandlungstisches mit maximal abgesenkter Tischplatte,
Fig. 8 eine der Fig. 7 entsprechende Vorderansicht des Großtierbehandlungstisches gemäß der vorliegenden Erfindung mit maximal angehobener Tischplatte,
Fig. 9 eine Draufsicht auf den erfindungsgemäßen Großtierbehandlungstisch mit verschiedenen Anbauteilen,
Fig. 10 eine Vorderansicht eines erfindungsgemäßen Großtierbehandlungstisches, der in den Anwendungsberiech eines Strahlerkopfes eines handelsüblichen Linearbeschleunigers verfahren ist,
Fig. 11 eine perspektivische Seitenansicht auf den Großtierbehandlungstisch und den Strahlerkopf des Linearbeschleunigers wie in Fig. 10 gezeigt,
Fig. 12 eine der Fig. 11 ähnliche perspektivische Ansicht von einer anderen Seite mit unterhalb der Tischplatte eines erfindungsgemäßen Großtierbehandlungstisches befindlichem Strahlerkopf.

### Detaillierte Beschreibung

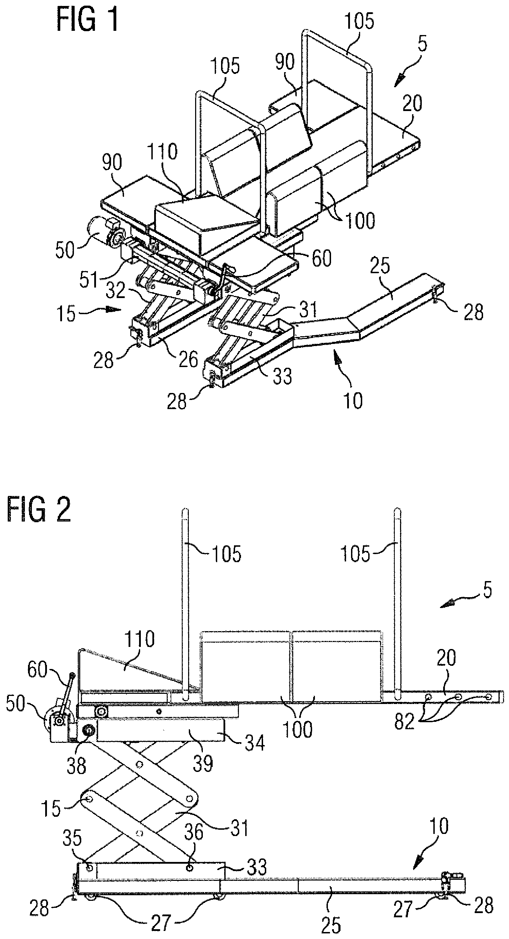

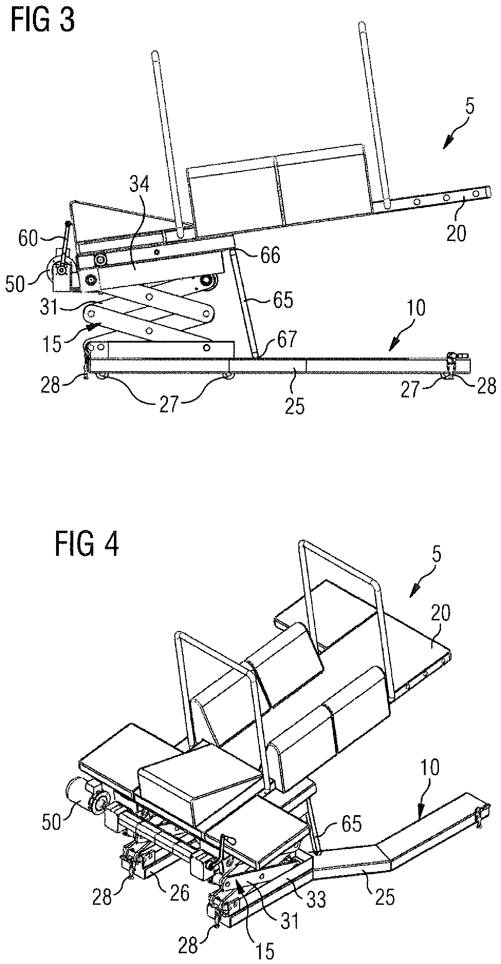

Eine erste beispielhafte Ausführungsform eines erfindungsgemäßen Großtierbehandlungstisches 5 ist in den Fig. 1 bis 4 gezeigt. Dieser Großtierbehandlungstisch 5 umfasst ein Grundgestell 10, eine Hubvorrichtung 15 und eine Tischplatte 20. Die Tischplatte 20 ist über die Hubvorrichtung 15 mit dem Grundgestell 10 verbunden. Wie insbesondere aus den Fig. 1 und 3 ersichtlich ist, sind diese drei Grundelemente eines erfindungsbemäßen Großtierbehandlungstisches 5 so zueinander angeordnet, dass sie in einer Seitenansicht eine asymmetrische oder insbesondere C-förmige Kontur bzw. Umriss haben.

Das Grundgestell 10 umfasst zwei voneinander beabstandete Fahrgestelllteile 25, 26, die jeweils mit Laufrollen 27 ausgestattet sind. Bei der hier gezeigten Ausführungsform sind an jedem Fahrgestellteil drei Laufrollen 27 vorhanden, eine Laufrolle 27 am hinteren Ende, eine Laufrolle 27 im mittleren Bereich und eine Laufrolle 27 im vorderen Bereich des jeweiligen Fahrgestellteils 25, 26. Die zwei Fahrgestellteile 25, 26 sind um eine lichte Innenweite voneinander beabstandet. Jedes Fahrgestellteil 25, 26 ist hier in einem Vorderbereich mit einer größeren lichten Innenweite ausgeführt als an einem Hinterbereich. Dadurch bilden die beiden einander spiegelverkehrt gegenüber liegenden Fahrgestellteile 25, 26 im Vorderbereich eine große lichte Innenweite, die größer ist als eine lichte Innenweite am Hinterende. Dies bewirkt eine größere Standfestigkeit des gesamten Großtierbehandlungstisches 5.

Die Fahrgestellteile 25, 26 sind hier im vorliegenden Ausführungsbeispiel dreiteilig ausgebildet, zwei geradlinige Teile, die durch ein nach außen schräggestelltes Mittelteil miteinander verbunden sind. An dem jeweiligen Hinterende eines Fahrgestellteils 25, 26 wie auch an einem vorderen Ende sind jeweils Festelleinrichtungen 28 angeordnet, die beispielsweise durch Betätigen mit dem Fuß ein Gummiteil auf dem Boden andrücken und so die Reibung erhöhen. Die Feststelleinrichtungen 27, 28 sind wie auf dem Markt üblich ausgebildet.

Die Hubvorrichtung 15 besteht hier aus zwei Scherenmechanismen 31, 32, die jeweils mit einem der Fahrgestellteile 25, 26 verbunden sind. So umfasst der rechte Scherenmechanismus 31 ein unteres Auflager 33 und ein oberes Auflager 34. Das untere Auflager 33 besteht hier aus einem hinteren unteren Lager 35, mit dem ein Teil des Scherenmechanismus drehbar verbunden ist. Ein Gleit- oder Rollenlager ist mit dem anderen unteren Teil des Scherenmechanismus 31 verbunden und gleitet bzw. rollt somit in Längsrichtung auf dem hinteren Teil des Fahrgestellteils 25.

Das obere Auflager 34 besteht aus einem oberen hinteren Lager 38 und einem oberen vorderen Gleit- oder Rollenlager 39. Das hintere obere Lager 38 ist mit dem einen Teil des Scherenmechanismus drehbar verbunden. Das obere vordere Gleit- oder Rollenlager rollt auf der Unterseite der Tischplatte bzw. einer dazwischen eingebrachten Platte ab.

Der tatsächliche detaillierte Aufbau und das Getriebe der Höhenverstellung sind hier nur angedeutet und teilweise in der Fig. 3 ersichtlich. Tatsächlich ist ein Motor 50 über eine Getriebewelle 51 mit einer hier nicht näher gezeigten Spindel verbunden, die den Abstand der beiden Teile des oberen Scherenmechanismus zueinander verändert und damit den Scherenmechanismus als solchen anhebt oder absenkt. Vorzugsweise sind beide Scherenmechanismen 31, 32 mit der gleichen Getriebewelle des Elektromotors verbunden, um so eine gleichmäßige Absenkung und Anhebung der Tischplatte 20 zu erzielen.

Wie insbesondere aus der Fig. 1 und 3 ersichtlich ist, ist zudem eine Kurbel 60 vorhanden, mit der die Getriebewelle 51 per Hand gedreht werden kann und somit die Höhe der Tischplatte 20 feinjustiert oder im Falle des Ausfalls des Elektromotors 50 per Hand in der Höhe veränderbar ist.

Wie insbesondere aus den Fig. 3 und 4 ersichtlich ist, ist die Tischplatte 20 neigbar. In der vorliegenden beispielhaften Ausführungsform eines erfindungsgemäßen Großtierbehandlungstisches 5 wird die Neigung durch Verstellen der Höhe der Tischplatte 20 mittels des Elektromotors 50 oder der Handkurbel 60 eingestellt. Hierzu ist ein Sperrbügel 65 an einem oberen Drehlagerpunkt 66 und einem unteren Drehlagerpunkt 67 mit einer Unterseite der Tischplatte 20 bzw. dem jeweiligen Fahrgestellteil 25, 26 drehbar verbindbar. Auf Grund der Starrheit des Sperrbügels 65 ist durch diese Verbindung der Abstand zwischen dem oberen Drehlagerpunkt 66 und dem unteren Drehlagerpunkt 67 festgelegt. Wenn nun, wie z.B. in Fig. 3 gezeigt, eine Absenkung der Tischplatte 20 erfolgt, wird der vordere Teil im Bereich des oberen Auflagers 34 nicht abgesenkt, sondern nur der hintere Teil. Dies kann deswegen erfolgen, da die Scherenmechanismen 31, 32 im vorderen Bereich eine Gleit- oder Rollenlagerung haben, wie es insbesondere in der Fig.3 ersichtlich ist, wo das vordere Rollenlager des Scherenmechanismus 31 aus dem oberen Auflager aufgrund der durch den Sperrbügel 65 bewirkten Neigung herausgetreten ist. Je stärker die Absenkung des Tisches 20 bei eingebrachtem Sperrbügel 65, desto größer wird die Neigung der Tischplatte 20.

Vorzugsweise ist der Sperrbügel 65 abnehmbar gestaltet oder zum Beispiel am oberen Drehlagerpunkt 66 verschwenkbar angebracht, so dass er bei Nichtbenutzung an der Unterseite er Tischplatte 20 verschwenkt fixiert werden kann und keine Sperrwirkung entfaltet. Falls eine Neigung gewünscht wird, wird der Sperrbügel 65 einfach nach untern verschwenkt und mit dem unteren Drehlagerpunkt 67 verbunden.

Es ist offensichtlich, dass der Sperrbügel 65 auch nur als Stab ausgebildet sein kann oder andere Gestalt haben kann.

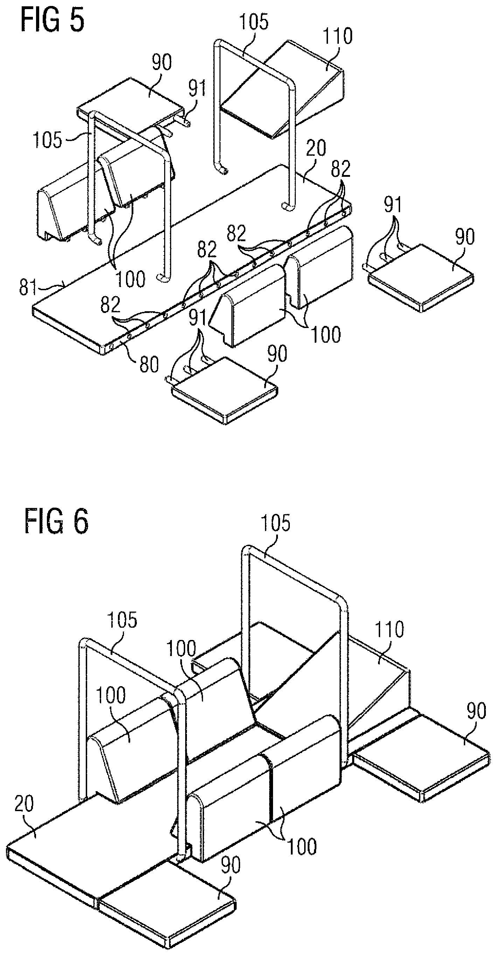

Im Folgenden wird insbesondere unter Bezugnahme auf die Fig. 5 und 6 näher auf die Ausgestaltung der Tischplatte 20 eingegangen. Die Tischplatte 20 ist hier aus CFK und/oder anderen strahlendurchlässigen Materialien ausgebildet und weist an den beiden Längsrändern 80, 81 eine Lochreihe 82 auf. Diese Löcher dienen als Stecköffnungen für komplementär ausgestaltete Stifte 91 von verschiedenen Anbauteilen wie Tischplattenverbreiterungen 90, Seitenwandteilen 100 und Fixierhalterungen 105 sowie eventuell eines Kopfteiles 110.

In der Fig. 6 ist die Tischplatte 20 mit den verschiedenen Anbauteilen 90, 100, 105 sowie dem Kopfteil 110 gezeigt. Hier sind diese Anbauteile 90, 100, 105 mittels ihrer Steckstifte an der Tischplatte eingesteckt. Durch die verschiedenen Lochreihen können individuell die Tischverbreiterungen etc. je nach Tier platziert werden. Die Fixierhalterungen 105 dienen dazu, eine Fixiermöglichkeit für Gliedmaßen von Pferden oder anderen Großtieren zu schaffen. Hier werden dann zum Beispiel einzelne Gliedmaßen mittels Bändern oder Gurten fixiert.

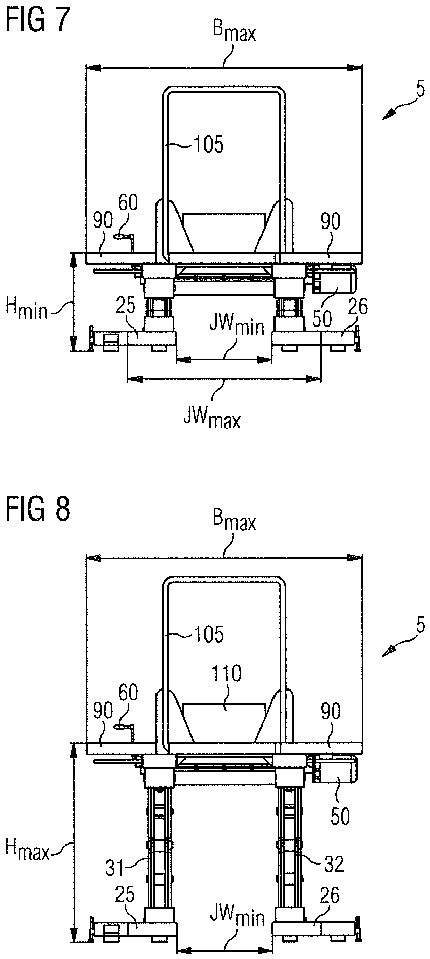

Aus den Fig. 7 bis 9 sind insbesondere die für das Überfahren des Großtierbehandlungstisches 5 über bestehende Tische von Computertomographiegeräten oder dergleichen gut ersichtlich. So beträgt die lichte Innenweite zwischen den beiden Fahrgestellteilen 25, 26 an der engsten Stelle im vorliegenden Ausführungsbeispiel 700 mm. Im vorderen Bereich beträgt die lichte Innenweite IWmax 800 mm. Die minimale Hubhöhe Hmin beträgt in dem vorliegenden Ausführungsbeispiel ca. 715 mm, die maximale Hubhöhe Hmax beträgt ca. 1470 mm. Die Hubhöhe Hmin bzw. Hmax ist diejenige Höhe, die von der Tischoberkante bis Boden gemessen ist. Mit den Tischverbreiterungen 90 beträgt die Tischbreite Tmax 2000 mm. Die Gesamtlänge des Behandlungstisches beträgt ca. 3000 mm und die Tischlänge L2 ca. 2000 mm. Die Fahrgestellteile 25, 26 haben eine Länge von ca. 2750 mm.

### Gewerbliche Anwendbarkeit

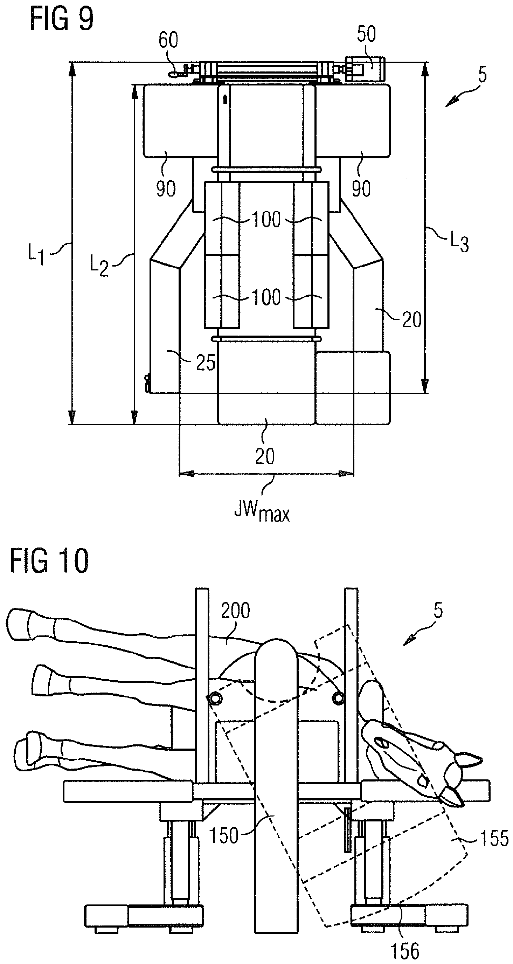

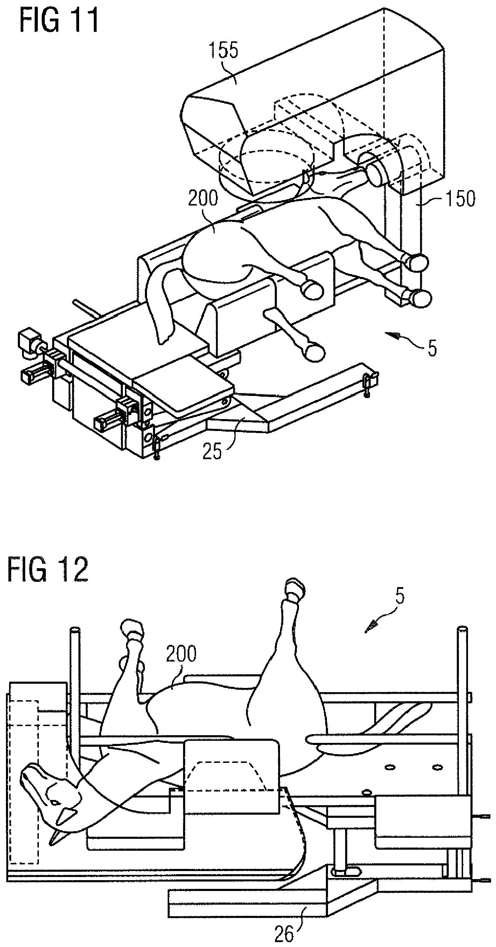

Aus den Darstellungen der Fig. 10 bis 12 ist insbesondere ersichtlich, wie ein erfindungsgemäßer Großtierbehandlungstisch 5 in Verbindung mit einem Linearbeschleuniger mit Strahlerkopf 155 einsetzbar ist. So ist hier der Beschleunigerkopf 155 des Linearbeschleunigers an einer Säule 150 frei drehbar gelagert. Durch die Ausgestaltung des Großtierbehandlungstisches 5 mit C-förmigem Umriss in der Seitenansicht und großer Öffnung im Vorderbereich des Fahrgestells mit den Fahrgestellteilen 25, 26 ist es ohne weiteres möglich, dass der Großtierbehandlungstisch 5 an den Linearbeschleuniger mit einem darauf gelagerten Pferd 200 oder einem anderen Tier herangefahren wird, auch wenn dieser einen eigenen Tisch aufweist. Darüber hinaus ist es ohne weiteres möglich, dass der Strahlerkopf 155, wie in Fig. 10 veranschaulicht, auch unterhalb des Behandlungstisches 5 rotieren kann, um bestimmte Behandlungen durchzuführen. Dabei streicht die Strahlerkopfaußenseite 156 nur knapp über dem Boden und den Vorderenden der beiden Fahrgestellteile 25, 26 vorbei.

Computertomographiegeräte, die mit einem erfindungsgemäßen Großtierbehandlungstisch kombinierbar sind, werden beispielsweise von den Firmen Siemens AG, General Electric, Philips und Toshiba vertrieben. Linearbeschleuniger, die zur Durchführung von Untersuchungen oder Behandlungen von auf einem erfindungsgemäßen Großtierbehandlungstisch geeignet sind, werden unter den Bezeichnungen: Varian Clinac (mit und ohne OBI), insbesondere der Bauart Clinac 2100, Clinac 2100C/D oder Clinac iX, True Beam, Elekta Synergy, Elekta Versa vertrieben.

Abschließend ist anzumerken, dass Großtierbehandlungstische der hier offenbarten Bauart statisch so ausgelegt sein müssen, dass Tiere mit einem Körpergewicht von bis zu 500 kg oder 800 kg oder insbesondere sogar bis 1.000 - 1.500 kg auf der Tischplatte gelagert werden können. Unter dem Begriff Großtierbehandlungstisch sind im vorliegenden Fall Behandlungstische zu subsummieren, die für Traglasten von mindestens 300 kg oder 500 kg ausgelegt sind, vorzugsweise aber Lasten von bis zu 1000 kg, 1300 kg oder sogar 1500 kg aufnehmen können.

Die hier verwendeten Begriffe "ungefähr", "etwa", "circa", "im Wesentlichen" oder "im Allgemeinen", die in Zusammenhang mit einem messbaren Wert wie beispielsweise einem Parameter, einer Menge, einer Form, einer zeitlichen Dauer oder dergleichen verwendet werden, oder auch explizite Wertangaben schließen Abweichungen oder Schwankungen von ± 10% oder weniger, vorzugsweise ± 5% oder weniger, weiter vorzugsweise ± 1% oder weniger und weiter vorzugsweise ± 0,1% des jeweiligen Wertes oder von dem jeweiligen Wert mit ein, sofern diese Abweichungen bei der Umsetzung der offenbarten Erfindung in die Praxis noch technisch sinnvoll sind. Es wird ausdrücklich darauf hingewiesen, dass der Wert, auf den sich der Begriff "ungefähr" bezieht, als solcher ausdrücklich und im Besonderen offenbart ist. Die Angabe von Bereichen durch Anfangs- und Endwerte umfasst all diejenigen Werte und Bruchteile dieser Werte, die von dem jeweiligen Bereich eingeschlossen sind, wie auch dessen Anfangs- und Endwerte.

## Patentansprüche

1. Großtierbehandlungstisch (5) mit:
- einem verfahrbaren Grundgestell (10), das in Längsachsenrichtung gesehen ein Vorderende und ein Hinterende aufweist und zumindest im Bereich des Vorderendes ein geteiltes Fahrgestell mit einem rechten Fahrgestellteil (25) und einem von dem rechten Fahrgestellteil (25) beabstandeten linken Fahrgestellteil (26) umfasst;
- einer Hubvorrichtung (15), die ein unteres Auflager (33) hat, mit dem die Hubvorrichtung (15) im Bereich des Hinterendes des Grundgestells (10) angeordnet ist, und ein oberes Auflager (34) besitzt;
- einer Tischplatte (20), die mit dem oberen Auflager (34) der Hubvorrichtung (15) derart verbunden ist, dass die Tischplatte (20) einen frei auskragenden Tischbereich hat und in einer Seitenansicht das Grundgestell (10), die Hubvorrichtung (15) und die Tischplatte (20) einen C-förmigen Umriss haben, so dass in Zusammenwirken mit dem geteilten Fahrgestell (25, 26) die Tischplatte (20) über einen Tisch eines üblichen Behandlungs- oder Diagnosegerätes wie z.B. ein Computertomographiegerät bewegbar ist und zusätzlich oder alternativ ein Strahlerkopf eines Linearbeschleunigers um den auskragenden Tischbereich herum rotierbar ist.

2. Großtierbehandlungstisch nach Anspruch 1, bei dem die Hubvorrichtung (15) in Längsrichtung gesehen jeweils einen linken Hubteil (31) und einen rechten Hubteil (32) umfasst, zwischen denen ein Freiraum vorhanden ist, der insbesondere ein Überfahren eines vorhandenen Behandlungstisches eines Computertomographiegerätes oder Linearbeschleunigers ermöglicht.

3. Großtierbehandlungstisch nach Anspruch 2, bei dem ein Motor (50) über ein mechanisches Getriebe (51) sowohl den rechten als auch den linken Hubteil (31, 32) in der Höhe verstellt.

4. Großtierbehandlungstisch nach Anspruch 2 oder 3, bei dem das rechte und linke Hubteil jeweils einen Scherenmechanismus (31, 32) umfassen.

5. Großtierbehandlungstisch (5) nach einem der voranstehenden Ansprüche, bei dem das Grundgestell (10) am Vorderende eine größere Breite aufweist als am Hinterende.

6. Großtierbehandlungstisch nach einem der voranstehenden Ansprüche, bei dem die Tischplatte (20) neigbar ist.

7. Großtierbehandlungstisch nach Anspruch 6, bei dem ein starres Sperrelement (60) an einem Ende verdrehbar mit dem oberen Auflager (34) und mit dem anderen Ende mit dem Grundgestell (10) verbindbar ist, so dass bei Absenken der Hubvorrichtung (15) die Tischplatte (20) sich automatisch neigt.

8. Großtierbehandlungstisch nach einem der voranstehenden Ansprüche, bei dem das obere Auflager (34) der Hubvorrichtung (15) an einem hinteren oberen Lager mit der Tischplatte (20) um eine Querachse verdrehbar gelagert und an einem vorderen oberen Lager mit der Tischplatte (20) gleitend gelagert ist.

9. Großtierbehandlungstisch nach einem der voranstehenden Ansprüche, bei dem das untere Auflager (33) der Hubvorrichtung (15) an einem hinteren Lager (35) mit dem Grundgestell (10) um eine Querachse verdrehbar gelagert ist und an einem vorderen Lager (36) an dem Grundgestell (10) gleitend gelagert ist.

10. Großtierbehandlungstisch (5) nach einem der voranstehenden Ansprüche, bei dem das rechte Fahrgestellteil (25) und das linke Fahrgestellteil (26) jeweils mit Laufrollen (27) versehen sind und vorzugsweise zumindest im Bereich des Vorderendes eine freie Öffnung mit einer lichten Innenweite (IW) von mindestens 700 mm definieren.

11. Großtierbehandlungstisch (5) nach einem der voranstehenden Ansprüche, bei dem das Grundgestell (10) wenigstens im Bereich des Vorderendes eine maximale Höhe (Hmax) über Boden von 150 mm aufweist.

12. Großtierbehandlungstisch nach einem der voranstehenden Ansprüche, bei dem die Tischplatte (20) auf eine Minimalhöhe (HMin) von 715 mm absenkbar ist.

13. Großtierbehandlungstisch nach einem der voranstehenden Ansprüche, bei dem die Tischplatte (20) auf eine Maximalhöhe (Hmax) von 1400 mm anhebbar ist.

14. Großtierbehandlungstisch nach einem der voranstehenden Ansprüche, bei dem die Tischplatte (20) an wenigstens einem Längsrand (80, 81) und/oder Seitenrand wenigstens eine Steckmöglichkeit (82) für ein oder mehrere Anbauteile wie Tischverbreiterungselemente (90), Seitenwandteile (100) oder Fixierhalterungen (105) aufweist.

15. Großtierbehandlungstisch nach einem der voranstehenden Ansprüche, bei dem zusätzlich zu dem Motor (50) zur Höhenverstellung der Tischplatte (20) eine per Hand zu betätigende Kurbel (60) zur Höhenverstellung der Tischplatte (20) vorhanden ist.
